# EUROPEAN PATENT APPLICATION

(11) **EP 2 460 797 A2**
(43) Date of publication of application: **06.06.2012**
(21) Application number: 11195861.7
(22) Date of filing: 30.11.2007
(51) Int. Cl.: C07D 223/16, C07C 217/56

(54) **Process for preparation of ivabradine hydrochloride**

(30) Priority: 30.11.2006 IN MU19662006
(62) Divisional of application: 07870531.6
(71) Applicant: Cadila Healthcare Limited, Ahmedabad 380 015 Gujarat (IN)
(72) Inventor: Kumar, Rajiv, 380 015 Gujarat (IN); Dwivedi, Shriprakash Dhar, 380 015 Gujarat (IN); Patel, Sunil Tribhovandas, 380 015 Gujarat (IN); Shah, Alpeshkumar Parvin Chandra, 380 015 Gujarat (IN)
(74) Representative: Clarke, Lionel Paul

(57) **Abstract**

The present invention relates to crystalline Ivabradine hydrochloride Form-I and a process for its preparation.

## Description

### FIELD OF INVENTION:

The present invention provides a process for preparation of Ivabradine hydrochloride of formula (I), its pharmaceutically acceptable salts, solvates and hydrates thereof. Ivabradine is chemically known as 3-{3-[{[(7S)-3,4-dimethoxybicyclo[4.2.0]octa-1,3,5-trien-7-yl]methyl}(methyl)amino]-propyl}-7,8-dimethoxy-1,3,4,5-tetrahydro-2H-3-benzazepin-2-one and its hydrochloride salt is useful in the treatment of myocardial ischaemia.

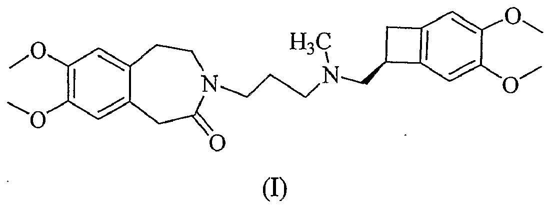

The present invention further provides a novel crystalline form of intermediate of Ivabradine having formula (II) and acid addition salt thereof. 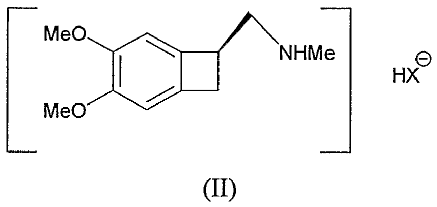 wherein HX = Camphor sulfonic acid

Ine one embodiment, the present invention provides a novel crystalline form of intermediate of formula (III) 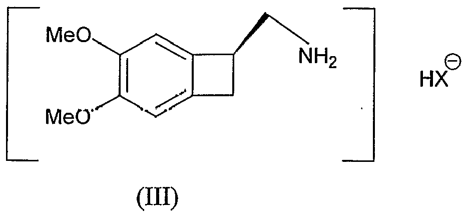 wherein HX = N-acetyl-L- gluatamic acid

The present invention also relates to the novel intermediate of formula (IV) for the preparation of Ivabradine or its pharmaceutically acceptable salt, solvates thereof.

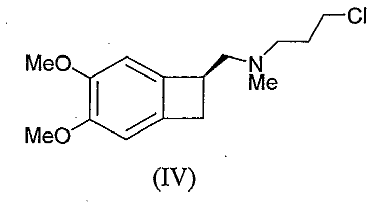

The present invention provides a the novel crystalline form of intermediate of Ivabradine having formula (V)

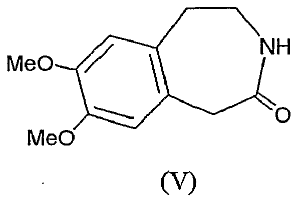

The present invention provides a novel intermediate for the preparation of Ivabradine hydrochloride with high yield and chiral purity.

### BACKGROUND OF THE INVENTION

Ivabradine hydrochloride, 3-{3-[{[(7S)-3,4-dimethoxybicyclo[4.2.0]octa-1,3,5-trien-7-yl]methyl}(methyl)amino]-propyl}-7,8-dimethoxy-1,3,4,5-tetrahydro-2H-3-benzazepin-2-one hydrochloride of formula (Ia) have very valuable pharmacological and therapeutic properties, especially bradycardic properties, making those compounds useful in the treatment or prevention of various clinical situations of myocardial ischaemia such as angina pectoris, myocardial infarct and associated rhythm disturbances, and also of various pathologies involving rhythm disturbances, especially supraventricular rhythm disturbances, and in the treatment of heart failure.

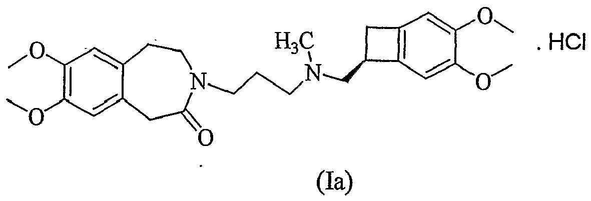

The preparation and therapeutic use of Ivabradine and salts thereof with a pharmaceutically acceptable acid, and more especially its hydrochloride, have been described in European Patent EP 0534859. This patent describes synthesis of Ivabradine hydrochloride by reacting the compound of formula (a) with the compound of formula (b): 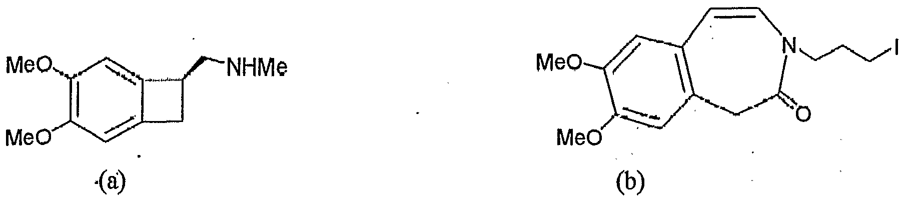 to give compound of formula (c), subsequent catalytic hydrogenation results in Ivabradine, which is further converted to its hydrochloride salt:

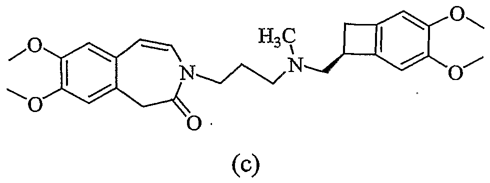

This disclosed process yields Ivabradine hydrochloride in a very low yield - less than 17% over the 3 steps as a whole.

Another process for preparing Ivabradine is disclosed in US 5296482. According to the process, (+) isomer of Ivabradine is treated with aqueous HCl and then recrystallization in acetonitrile leads to the formation of Ivabradine hydrochloride salt having M.P. 135-140°C.

US patent no. 7,176,197 B2 discloses a process for preparation of α - crystalline form of Ivabradine hydrochloride which comprises reacting 3-[2-(1,3-dioxolan-2-yl) ethyl]-7,8-dimethoxy-1,3-dihydro-2H-3-benzazepin-2-one with ethanol and then followed by addition of (7S)-[3,4-dimethoxybicyclo[4.2.0] octa-1,3,5-trien-7-yl]-N-methylmethan-amine hydro chloride with ethanol and water and final crystallization from toluene / 1-methyl-2-pyrrolidinone mixture affords α - crystalline form of Ivabradine hydrochloride.

US 2006/0194962 A1 discloses a process for preparation of the β-crystalline form of Ivabradine hydrochloride, which process is **characterized in that** a mixture of Ivabradine hydrochloride and water or a mixture of Ivabradine hydrochloride, isopropanol and water is heated until dissolution is complete and is then progressively cooled until crystallization is complete, and the crystals formed are collected.

US 2006/0194963 A1 also discloses a process for preparation of the γ-crystalline form of Ivabradine hydrochloride, which process is **characterized in that** a mixture of Ivabradine hydrochloride and 2-ethoxyethanol, a mixture of Ivabradine hydrochloride, 2-ethoxyethanol and water, or a mixture of Ivabradine hydrochloride, ethanol and water is heated until dissolution is complete and is then cooled until crystallization is complete, and the product is collected by filtration.

US 2006/0194965 A1 discloses a process for the preparation of βd-crystalline form of Ivabradine hydrochloride, which process is **characterized in that** a mixture of Ivabradine hydrochloride and water or a mixture of Ivabradine hydrochloride, isopropanol and water is heated until dissolution is complete and is then progressively cooled until crystallization is complete and the crystals thereby formed are collected and dehydrated.

US 2006/01945964 A1 relates also to a process for preparation of the γd-crystalline form of Ivabradine hydrochloride, which process is **characterized in that** a mixture of Ivabradine hydrochloride and 2-ethoxyethanol, a mixture of Ivabradine hydrochloride, 2-ethoxyethanol and water, or a mixture of Ivabradine hydrochloride, ethanol and water is heated until dissolution is complete and is then cooled until crystallization is complete, and the crystals obtained are collected by filtration and dehydrated.

US 2007/0082886 discloses [delta]d-crystalline form of Ivabradine hydrochloride and a process for the preparation of the [delta]d-crystalline form of Ivabradine hydrochloride, wherein acetonitrile or a mixture of acetonitrile and water is preheated, Ivabradine hydrochloride is added, the solution obtained is allowed to cool at room temperature, held at room temperature until crystallisation is complete, and the crystals formed are dehydrated.

### OBJECT OF INVENTION:

An object of the present invention is to provide a process for preparing Ivabradine of formula (I).

Another object of the present invention is to provide a novel intermediate of formula (IV) for the preparation of Ivabradine hydrochloride.

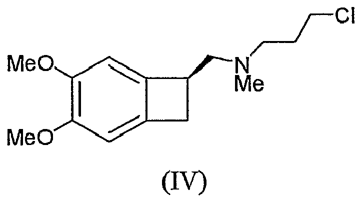

Yet another object of the present invention is to provide a novel crystalline form of (1S)-4,5-dimethoxy-1-(methylamine)-benzocyclobutane -N-acetyl-L- gluatamic acid.

Still another object of the present invention is to provide a novel crystalline form of (1S)-4,5-dimethoxy-1-(methylamiomethyl)-benzocyclobutane camphor sulfonic acid.

Yet another object of the present invention is to provide a crystalline form of 7,8-dimethoxy-1,2,3,4-tetrahydro-2H-3-benzapin-2-one.

### SUMMARY OF THE INVENTION:

The present invention provides a process for preparing Ivabradine of formula (I) or its phamacetically acceptable salts, solvates or hydrates thereof via novel intermediate of formula (IV).

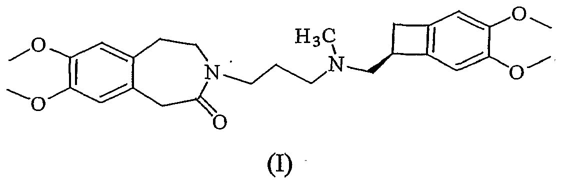

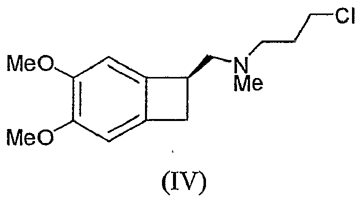

The present invention further provides a novel crystalline, amorphous, hydrate and solvate forms of intermediate of Ivabradine hydrochloride having formula (II) and acid addition salt thereof. 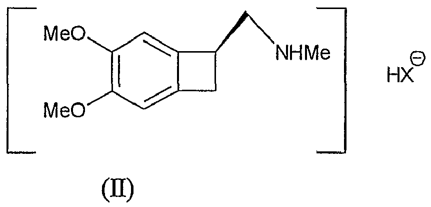 wherein HX = Camphor sulfonic acid

One embodiment of the present invention provides a novel crystalline form of intermediate of formula (III) 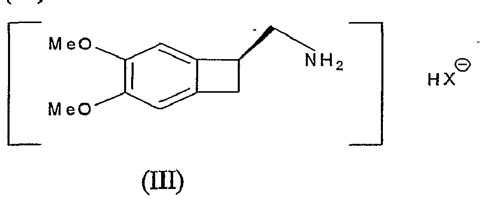 wherein HX = N-acetyl-L- gluatamic acid

The present invention relates to novel intermediate of formula (IV) and its process for preparation.

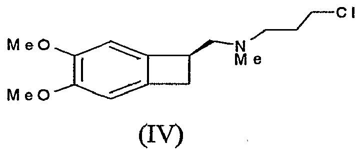

The present invention provides a the novel crystalline form of intermediate of Ivabradine having formula (V)

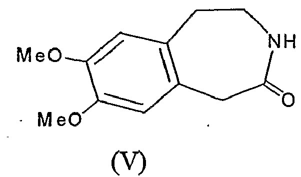

The present invention provides a novel intermediate for the preparation of Ivabradine hydrochloride with high yield and chiral purity.

### DETAILED DESCRIPTION OF THE INVENTION:

The present invention provides a process for preparing Ivabradine of formula (I) or its pharmaceutically acceptable salts, solvates, hydrate thereof, 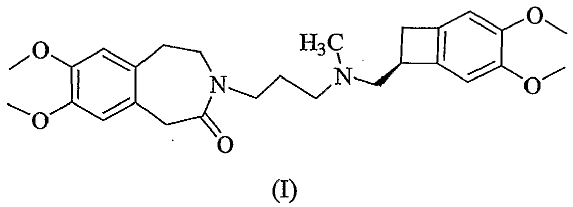 by reacting 3-chloro-N-{[(7S)-3,4-dimethoxybicyclo[4.2.0]octa-1,3,5-trien-7-yl]methyl}-N-methylpropan-1-amine of formula (IV), 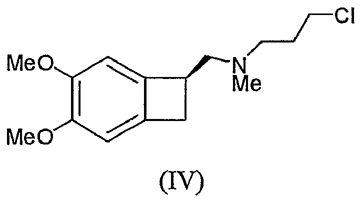 with 7,8-Dimethoxy-1,3,4,5-tetrahydro-benzo[d]azepin-2-one of formula (V) or its alkaline salt 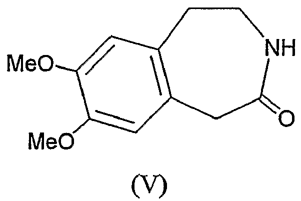 in presence of base in suitable solvent to provide Ivabradine of formula (I). This can be subsequently converted to its pharmaceutically acceptable salts, solvate or hydrate thereof

The condensation reaction of compound of formula (IV) with compound of formula (V) is preferably carried out in presence of base selected from sodium hydroxide, potassium hydroxide, sodium carbonate, potassium carbonate, sodium methoxide, sodium ethoxide, potassium tert-butoxide, sodium hydride and the like. The preferred base is potassium carbonate or potassium tert-butoxide.

More preferably, compound of formula (V) is treated with alkali metal hydroxide, carbonate or alkoxide to form alkali metal salt of formula (V), which is further reacted with compound for formula (IV) to provide Ivabradine.

The reaction is preferably carried out in suitable solvent. The solvent system is preferably selected so as to facilitate the condensation reaction and to allow subsequent separation of Ivabradine. Advantageously, both compound of formula (IV) and the compound of formula (V) are dissolvable, at least partly, in the solvent system, at least at elevated temperatures. In the process, a mixture, slurry, or solution of compound of formula (V) and a solvent may be contacted with a compound of formula (IV), or conversely, a mixture, slurry, or solution of compound of formula (IV) and a solvent may be contacted with compound of formula (V). In another embodiment, both partners may be combined with a solvent system prior to being contacted together, whereby the solvent system used for compound (IV) may be identical with or different from the solvent system used for the compound (V). The solvent system can be comprised of a single solvent or a mixture of solvents.

Suitable solvents include polar protic or aprotic solvent selected from a lower alcohol (C₁- C₆) such as methanol, ethanol, isopropanol, n-propanol, n-butanol, iso-butanol, tert-butanol; ether such as tetrahydrofuran, diethyl ether, diisopropyl ether, dioxane; sulfoxide such as sulfolane or dimethyl sulfoxide, amides such as dimethyl formamide, dimethyl acetamide, acetonitrile and the like or mixtures thereof.

The temperature of contact of both the compounds in the solvent system is from ambient to the boiling point of the solvent system, with elevated temperatures, but generally less than the boiling point, being preferred.

In a preferred embodiment of the present invention, there is provide a process for preparing Ivabradine of formula (I) or its pharmaceutically acceptable salt, preferably hydrochloride comprising reaction of 3-chloro-N-{[(7S)-3,4-dimethoxybicyclo[4.2.0]octa-1,3,5-trien-7-yl]methyl}-N-methylpropan-1-amine of formula (IV) with 7,8-Dimethoxy-1,3,4,5-tetrahydro-benzo[d]azepin-2-one of formula (V) in presence of base selected from potassium tert-butoxide in dimethylsulfoxide to provide Ivabradine of formula (I).

The present invention also provides 3-chloro-N-{[(7S)-3,4-dimethoxybicyclo [4.2.0] octa-1,3,5-trien-7-yl]methyl}-N-methylpropan-1-amine of formula (IV), a novel intermediate useful for the preparation of Ivabradine.

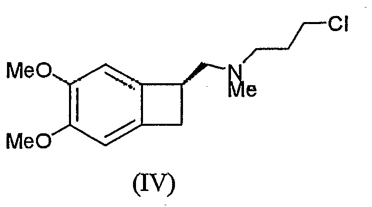

The present invention further provides a process for preparation of compound of formula (IV), which comprises reacting 1-[(7S)-3,4-dimethoxybicyclo[4.2.0]octa-1,3,5-trien-7-yl]-N-methylmethanamine of formula (VI) with 1-Bromo-3-chloropropane of formula (VII) in presence of base to form the novel compound of formula (IV)

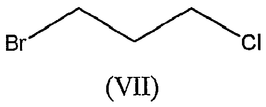

The reaction is preferably carried out in presence of base selected from sodium hydroxide, potassium hydroxide, sodium bicarbonate, and potassium bicarbonate. The preferred base potassium bicarbonate. The reaction can be preferably carried out in presence of solvent selected from lower alcohol (C₁- C₆) such as methanol, ethanol, isopropanol, n-propanol, n-butanol, iso-butanol, tert-butanol; ether such as tetrahydrofuran, diethyl ether, diisopropyl ether, dioxane; sulfoxide such as sulfolane or dimethyl sulfoxide, amides such as dimethyl formamide, dimethyl acetamide and the like. Preferably, reaction is carried out in neat condition at room temperature.

The present invention provides novel crystalline form of (1S)-4,5-dimethoxy-1-(methylaminomethyl)-benzocyclobutane camphor sulfonate of formula (II), which is one of the intermediate for the preparation of Ivabradine. 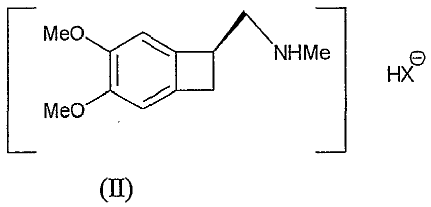 wherein HX = Camphor sulfonic acid

The novel crystalline salt of (1S)-4,5-dimethoxy-1-(methylaminomethyl)-benzocyclobutane-camphor sulphonic acid is characterized by its X-ray powder diffraction pattern having peaks at 7.1, 8.7, 10.9, 14.0, 14.4, 15.0, 17.5, 18.6, 18.9, 20.9, 21.2, 21.4, 22.1, 22.5, 23.9, 24.2, 24.5, 24.9, 25.6, 26.0, 26.6, 27.2, 27.9, 28.5, 29.3, 30.9, 31.4, 31.8, 32.2, 33.6, 34.3, 35.5, 36.5, 37.3, 37.8 +/- 0.2 two theta values.

The crystalline salt of (1S)-4,5-dimethoxy-1-(methylaminomethyl)-benzocyclobutane-camphor sulphonic acid is further characterized by differential scanning calorimetry having endotherm at 165-166°C

According to another embodiment of the present invention, there is provided a process fur preparing novel crystalline form of (1S)-4,5-dimethoxy-1-(methylaminomethyl)-benzocyclo butane camphor sulfonate of formula (II), which comprises reaction of 4,5-dimethoxy-1-(aminomethyl)benzocyclobutane with camphor sulfonic acid in suitable organic solvent and then recrystallized in a suitable organic solvent to form the novel crystalline form. Novel crystalline form of this acid addition salt by recrystallization in different solvents like alcohol selected from methanol, ethanol, n-propanol, isopropanol, n-butanol, isobutanol, tert-butanol, ketones selected from acetone, methyl ethyl ketone, methyl isobutyl ketone and the like or mixtures therof.

One of the embodiments of the present invention comprises a novel crystalline form of intermediate of formula (III) 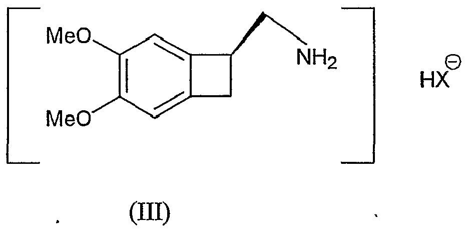 wherein HX = N-acetyl-L- gluatamic acid

The present invention provides a crystalline (1S)-4,5-dimethoxy-1-(methylamine)-benzocyclobutane N-acetyl-L- gluatamic acid characterized by X-ray powder diffraction pattern having peaks at 8.3, 9.7, 10.7, 12.5, 14.5, 16.0, 16.7, 17.9, 18.7, 19.2, 19.6, 20.4, 21.2, 21.7, 22.7, 23.7, 24.2, 24.6, 25.7, 26.2, 26.7, 28.0, 29.4, 29.8, 30.8, 31.5, 32.4, 33.2, 34.7, 3 6.0, 36.9, 37.5, 38.3, 39.1 +/- 0.2 two theta values.

Crystalline salt of (1S)-4,5-dimethoxy-l-(methylamine)-benzocyclobutane-N-acetyl-L- gluatamic acid is further characterized by differential scanning calorimetry endotherm at 142-143°C.

The present invention relates to the preparation of novel crystalline salt of (1S)-4,5-dimethoxy-1-(methylamine)-benzocyclobutane N-acetyl-L- gluatamic acid of formula (III), which comprises reaction of 1-(3,4-dimethoxybicyclo[4.2.0]octa-1,3,5-trien-7-yl)methan amine with N-acetyl glutamic acid in suitable organic solvent and then recrystallized in a suitable organic solvent to form the novel crystalline form. Novel crystalline form of this acid addition salt by recrystallization in different solvents like alcohol, ketones, esters such as methanol, ethanol, n-propanol, isopropanol, n-butanol, isobutanol, tert-butanol, acetone, methyl ethyl ketone, methyl isobutyl ketone or mixtures thereof.

Thus obtained (1S)-4,5-dimethoxy-1-(methylamine)-benzocyclobutane N-acetyl=L-gluatamic acid of formula (III) is further converted to 1-[(7S)-3,4-dimethoxybicyclo[4.2.0]octa-1,3,5-trien-7-yl]methanamine formula (VIII),which upon 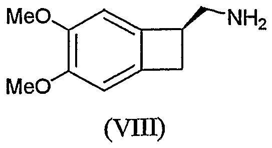 methylation gives 1-[(7S)-3,4-dimethoxybicyclo[4.2.0]octa-1,3,5-trien-7-yl]-N-methylmethanamine (VI) of formula

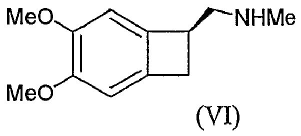

According to another embodiment of the present invention, there is provided a novel crystalline form of 7,8-dimethoxy-1,2,3,4-tetrahydro-2H-3-benzapin-2-one of formula (V)

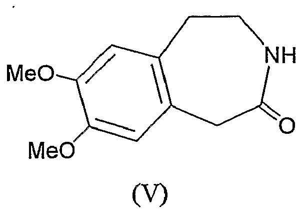

The invention provides crystalline form of 7,8-dimethoxy-1,2,3,4-tetrahydro-2H-3-benzapin-2-one having X-ray powder diffraction pattern with peaks at 4.6, 9.2, 11.3, 11.9, 12.9, 13.2, 13.9, 14.9, 15.4, 16.1, 16.6, 17.1, 18.6, 19.7, 20.1, 20.5, 21.2, 21.8, 22.5, 23.3, 24.5, 25.7, 26.7, 27.0, 28.1, 28.8, 29.9, 30.2, +/- 0.2 two theta values. The crystalline form of 7,8-dimethoxy-1,2,3,4-tetrahydro-2H-3-benzapin-2-one has differential scanning calorimeter at 196-197°C.

The present invention further provides a process for the preparation of crystalline form of 7,8-dimethoxy-1,2,3,4-tetrahydro-2H-3-benzapin-2-one having X-ray powder diffraction pattern with peaks at 4.6, 9.2, 11.3, 11.9, 12.9, 13.2, 13.9, 14.9, 15.4, 16.1, 16.6, 17.1, 18.6, 19.7, 20.1, 20.5, 21.2, 21.8, 22.5, 23.3, 24.5, 25.7, 26.7, 27.0, 28.1, 28.8, 29.9, 30.2, +/- 0.2 two theta values, which comprises treating 7,8-dimethoxy-1,2,3,4-tetrahydro-2H-3-benzapin-2-one with alkyl acetate and preferably ethyl acetate.

The invention provides a novel crystalline forms of Ivabradine hydrochloride, herein after designated as "Form- I" having characteristic X-ray powder diffraction pattern with peaks at 8.6, 9.1, 11.7, 14.6, 17.2, 18.2, 21.5, 23.9, 26.4 and 27.0 +/- 0.2 two theta values. The Form I of Ivabradine hydrochloride is further characterized by X-ray powder diffraction peaks at 11.2, 15.3, 19.6, 20.3, 21.0, 22.2, 22.7, 27.7, 29.4 +/- 0.2 two theta values.

According to an embodiment of the present invention, the process for preparing Form I of Ivabradine hydrochloride involves dissolving Ivabradine freebase in suitable organic solvent and treated with HCl to form Ivabradine hydrochloride, which is subsequently recrystallized from acetonitrile to obtain Ivabradine hydrochloride Form-I.

The reaction of Ivabradine base with HCl is preferably carried out in suitable solvent. The solvent system is preferably selected so as to facilitate the salt reaction and to allow subsequent separation of the resulting hydrochloride salt. Advantageously, both Ivabradine and the hydrochloric acid are dissolvable, at least partly, in the solvent system, at least at elevated temperatures. In the process, a mixture, slurry, or solution of Ivabradine and a solvent may be contacted with a hydrochloric acid, or conversely, a mixture, slurry, or solution of hydrochloric acid and a solvent may be contacted with Ivabradine. In another embodiment, both partners may be combined with a solvent system prior to being contacted together, whereby the solvent system used for hydrochloric acid may be identical with or different from the solvent system used for the Ivabradine. The solvent system can be comprised of a single solvent or a mixture of solvents. When two or more solvents are used, a two-phase reaction scheme may be used wherein the Ivabradine and hydrochloric acid are primarily reacted in one phase and the resulting Ivabradine hydrochloric acid compound is primarily present in the other phase due to, inter alia, solubility differences, etc. Suitable solvents include a lower alcohol (C₁- C₄) such as methanol, ethanol, isopropanol, n-propanol, n-butanol, iso-butanol, tert-butanol; ester such as ethyl acetate, isopropyl acetate, butyl acetate, iso-butyl acetate; ketone such as acetone, methyl ethyl ketone, methyl tert-butyl ketone; ether such as tetrahydrofuran, diethyl ether, Diisopropyl ether, dioxane and the like.

The following examples describe the invention and are non-limiting in nature.

### BRIEF DETAILS OF DRAWINGS

**Fig.I** is a characteristic X-ray powder diffraction pattern for Ivabradine Hydrochloride Form-I..
**Fig. II** is a characteristic differential scanning calorimetry thermogram of Ivabradine Hydrochloride Form-I.
**Fig. III** is a characteristic X-ray powder diffraction pattern for crystalline form of (1S)-4,5-dimethoxy-1-(methylamine)-benzocyclobutane N-acetyl-L- gluatamic salt.
**Fig. IV** is a characteristic differential scanning calorimetry thermogram of crystalline form of (1S)-4,5-dimethoxy-1-(methylamine)-benzocyclobutane N-acetyl-L- gluatamic salt.
**Fig. V** is a characteristic thermal gravimetric analysis of crystalline form of (1S)-4,5-dimethoxy-1-(methylamine)-benzocyclobutane N-acetyl-L- gluatamic salt.
**Fig. VI** is a characteristic X-ray powder diffraction pattern for crystalline form of (1S)-4,5-dimethoxy-1-(methylaminomethyl)-benzocyclobutane-camphor sulphonate salt.
**Fig. VII** is a characteristic differential scanning calorimetry thermogram of crystalline form of (1S)-4,5-dimethoxy-1-(methylaminomethyl)-benzocyclobutane-camphor sulphonate salt.
**Fig. VIII** is a characteristic X-ray powder diffraction pattern for crystalline form of 7,8-dimethoxy-1,2,3,4-tetrahydro-2H-3-benzapin-2-one.
**Fig. IX** is a characteristic differential scanning calorimetry thermogram of crystalline form of 7,8-dimethoxy-1,2,3,4-tetrahydro-2H-3-benzapin-2-one.

### WORKING EXAMPLES:

### 1) Preparation of 3-(Chloro-propyl)-(3,4-dimethoxy-bicyclo[4.2.0]octa-1(6),2,4-trien-7-ylmethyl)-methyl-amine

In a 1 liter Round bottom flask equipped with over head stirrer, thermometer pocket with thermometer and addition funnel with nitrogen gas inlet, (1S)-4,5-dimethoxy-1-(methyl aminomethyl)-benzocyclobutane (100 g, 0.48 mole), [compound is obtained after breaking the camphorsulphonic acid salt of (1S)-4,5-dimethoxy-1-(methyl aminomethyl)-benzocyclo butane with sodium hydroxide], potassium carbonate (99.9 g, 0.72 mole) and 1-bromo-3-chloropropane (300 ml) was added and stirred for 5 Hr at room temperature. Further, MDC (300 ml) and water (300 ml) was added and stirred for 15 min. the layers were separated and MDC layer was washed with 5.0 % HCl (3 x 100 ml). To the aqueous layer pH was adjusted to pH 9-10 with liquor ammonia. Further, MDC (500 ml) was added and stirred for 15 min and layers were separated in similar manner as above. Finally, MDC layer was washed with water (2 x 100 ml) and dried over anhydrous sodium sulfate and solvent was distilled out to afford the title compound 3-(Chloro-propyl)-(3,4-dimethoxy-bicyclo[4.2.0]octa-1(6),2,4-trien-7-ylmethyl)-methyl-amine.

### 2) Preparation of Ivabradine Base

In a 1 liter Round bottom flask equipped with over head stirrer, thermometer pocket with thermometer and addition funnel with nitrogen gas inlet, 7,8-Dimethoxy-1,3,4,5-tetrahydro-benzo[d]azepin-2-one (81.86 g, 0.37 mole) was added followed by the addition of DMSO (160 ml) at room temperature. Further, Potassium-tert-butoxide (47.45 g, 0.42 mole) was added and a clear solution was observed and solid separates out within 5 min. The reaction mixture was stirred for 1 Hr and after stirring for 1 Hr, 3-(Chloro-propyl)-(3,4-dimethoxy-bicyclo[4.2.0]octa-1(6),2,4-trien-7-ylmethyl)-methyl-amine (110 g, 0.35 mole) was added being dissolved in DMSO (150 ml) within 1 Hr at 25-30°C. The reaction mixture was stirred for 4-5 Hrs at same temperature condition. After completion of the reaction the reaction mass was added to chilled water (1.5 L) and stirred for 15 min. After stirring the layers were separated and the organic layer was dried on sodium sulfate and solvent was completely distilled out to afford the title compound as Ivabradine base.

### 3) Preparation of Ivabradine Hydrochloride

In a 1 liter Round bottom flask equipped with over head stirrer, thermometer pocket with thermometer and addition funnel with nitrogen gas inlet, Ivabradine Base (100g, 0.21 mole) was added followed by the addition of acetonitrile (300 mL) at 20°C. Further, to the reaction mixture IPA HCl was added drop wise till the pH reaches to 1-2 and the solid was appeared. The reaction mass was cooled to 15-20°C and stirred for 12 Hr at 20°C. After stirring solid was formed and the solid was filtered and washed with acetonitrile under nitrogen atmosphere. Finally, the solid was recrystallized with acetonitrile under reflux condition for 2 Hrs and the solid was filtered and washed with acetonitrile to afford crystalline form of Ivabradine hydrochloride.

### 4) Preparation of Glutamte salt of (1S)-4,5-dimethoxy-1-(methylamine)-benzocyclobutane

In a 1 liter Round bottom flask equipped with over head stirrer, thermometer pocket with thermometer and addition funnel with nitrogen gas inlet (1S)- 4,5-dimethoxy-1-(methyl amine)-benzocyclobutane (100 g, 0.51 mole) and ethanol (2 L) was added. Further, N-acetyl glutamic acid (98.0 g, 0.51 mole) was added and reaction mass was stirred for 6 Hrs at 80°C. The reaction mass was cooled to 25-30°C and further cooled to 20°C and stirred for 8 Hrs and solid comes out. The solid mass was washed with ethanol (30 mL). The wet solid was crystallized with ethanol (800 mL). The reaction mass was cooled to 15-20°C and stirred for 8 Hrs and again the solid was washed with ethanol (40 mL) and dried in hot air oven for 5 Hrs at 60°C. The solid was again recrystallized with ethanol. To the solid mass water was added and further NaOH solution (230 mL) was added and stirred for 15 min. at 20°C. Further to the reaction mass MDC (370 mL) was added and the layers were separated out. MDC layer was washed with water and dried on sodium sulfate and MDC was distilled out completely under vacuum to afford the title compound as Glutamate salt of (1S)-4,5-dimethoxy-1-(methylaminomethyl)-benzocyclobutane.

### 5) Preparation of Camphor sulphonic acid salt of (1S)-4,5-dimethoxy-1-(methyl aminomethyl)-benzocyclobutane

In a 1 liter Round bottom flask equipped with over head stirrer, thermometer pocket with thermometer and addition funnel with nitrogen gas inlet (1S)-4,5-dimethoxy-1-(methylaminomethyl)-benzocyclobutane (100 g, 0.48 mole) and (+) Camphor sulphonic acid (500 mL) and IPA (500 mL) was added and refluxed for 6 Hrs. The reaction mixture was slowly cooled to room temperature and stirred for 8 Hrs. After stirring the solid washed with IPA and dried in oven at 60°C for 6 Hrs. The solid was added into water (5 L) and basified with NaOH till the pH becomes 12. The reaction was stirred well and extracted with MDC. Finally the solid was dried on sodium sulfate and MDC was distilled out completely to afford Camphor sulphonic acid salt of (3,4-dimethoxy-bicyclo[4.2.0]octa-1(6),2,4-trien-7-ylmethyl)methylamine.

### 6) Preparation of crystalline salt of 7,8-Dimethoxy-1,3,4,5-tetrahydro-benzo[d]azepin-2-one

In a 1 liter Round bottom flask equipped with over head stirrer, thermometer pocket with thermometer and addition funnel with nitrogen gas inlet, 7,8-Dimethoxy-2-oxo-1,2,4,5-tetrahydro-benzo[d]azepine-3-carboxylic acid tert-butyl ester (100 g, 0.32 mole) and MDC (350 mL) was added. The reaction mixture was stirred for 15 min. at room temperature. Further the temperature was maintained upto 0-5°C. To the reaction mixture TFA (125 mL) was added drop wise within 2 Hrs at 0-5°C and stirred for 3 Hrs at 0-5°C. To the reaction mixture MDC (2 L) was added and the reaction mass was added into water (500 mL). The layers were separated and to the MDC layer sodium bicarbonate solution (620 mL) was added and again MDC layer was separated out and dried on sodium sulfate and solvent was distilled out completely. Finally to the residue ethyl acetate (250 mL) was added and stirred at 0-5°C for 4 Hrs. The solid was filtered at 0-5°C and finally washed with ethyl acetate to afford the crystalline form of 7,8-Dimethoxy-1,3,4,5-tetrahydro-benzo[d]azepin-2-one.

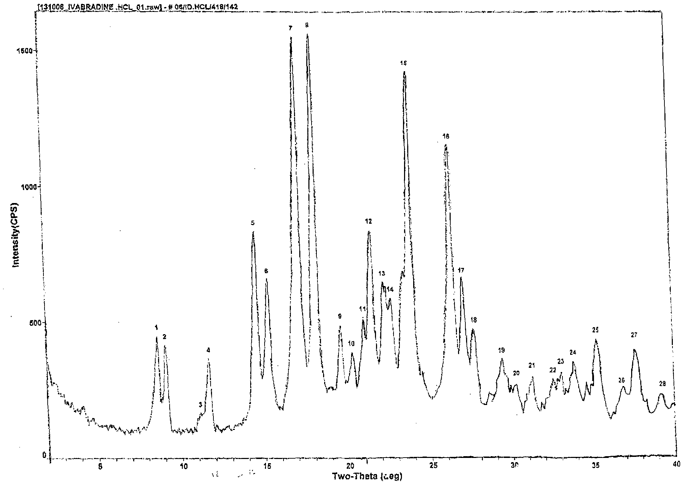

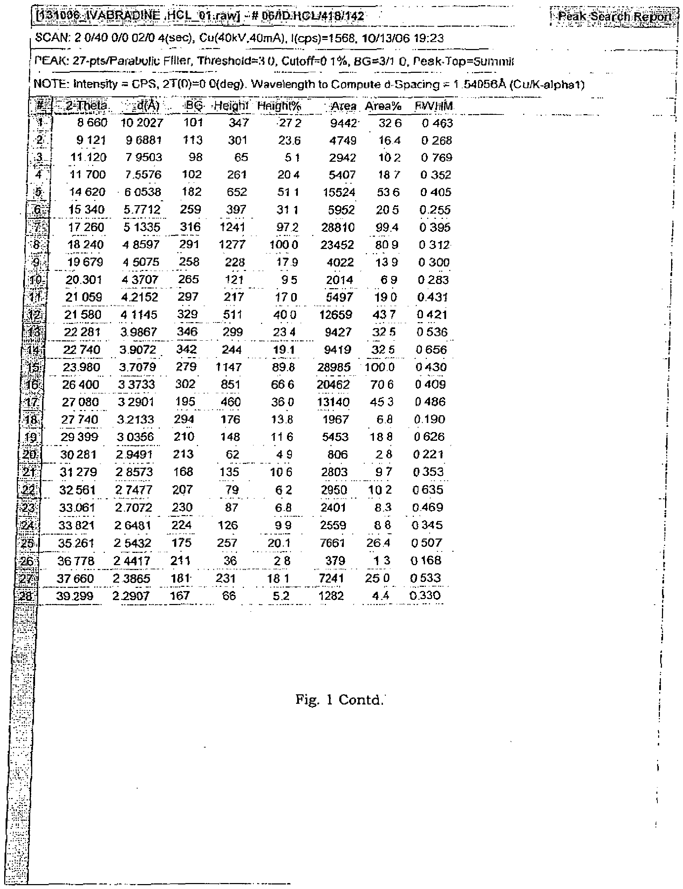

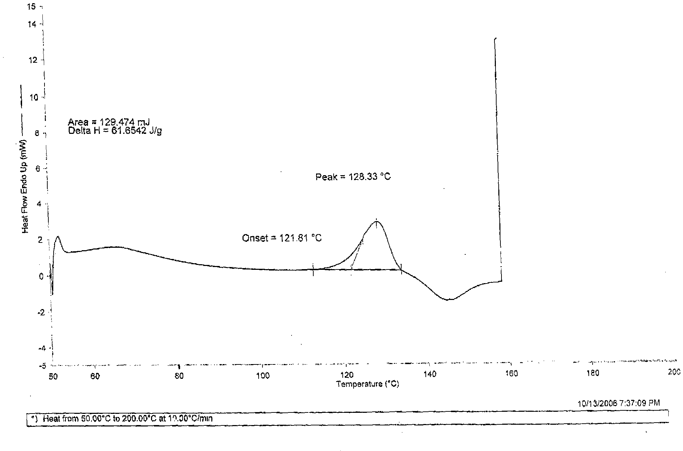

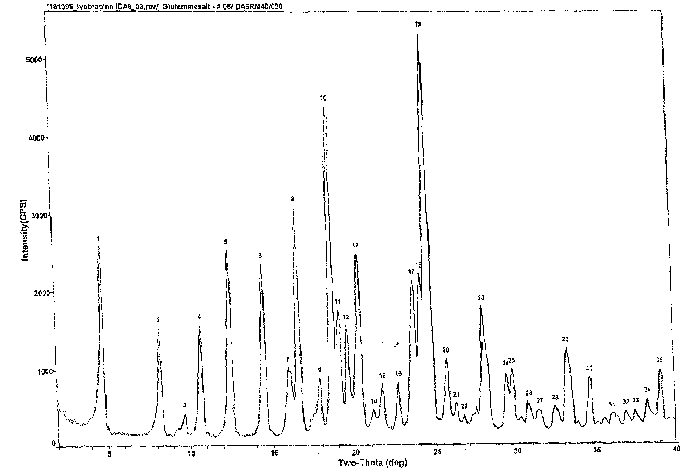

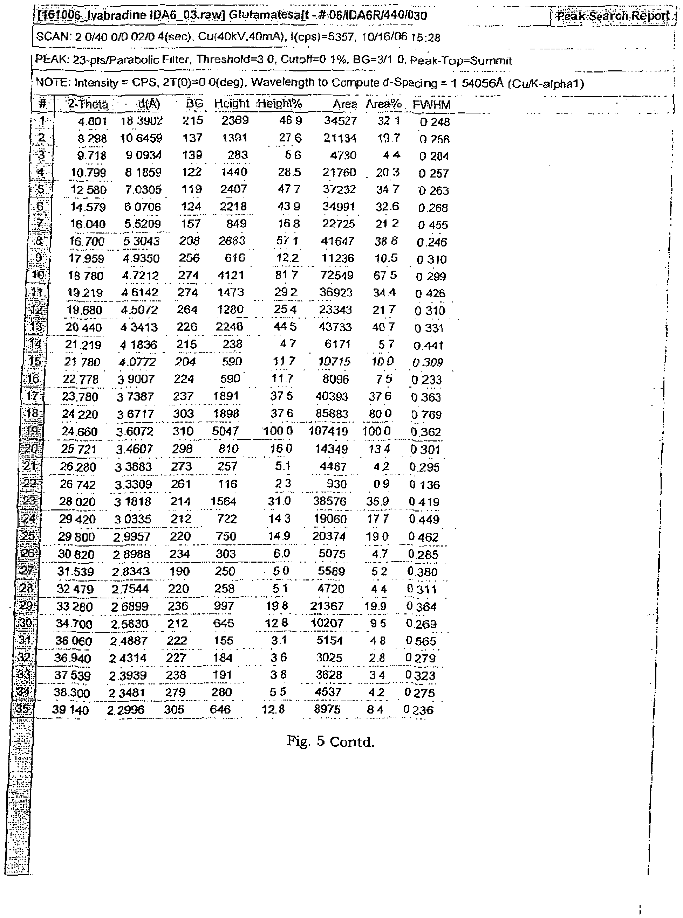

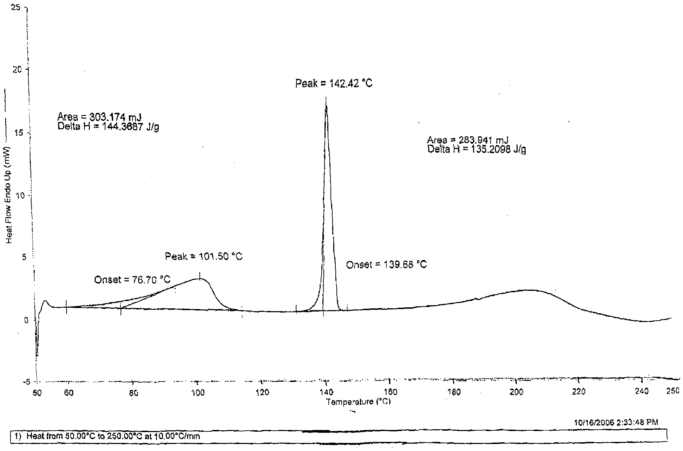

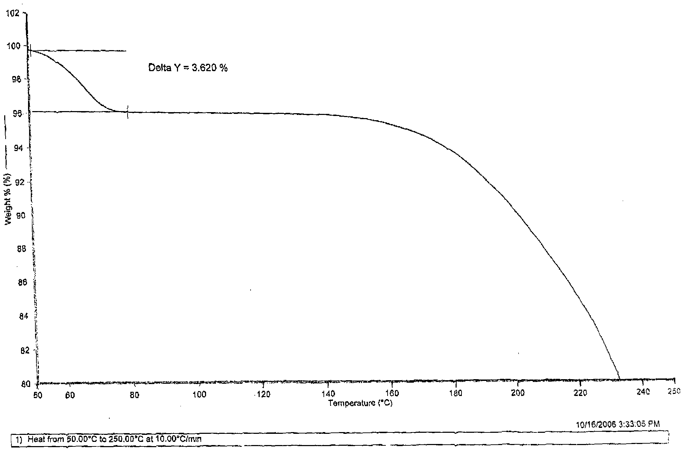

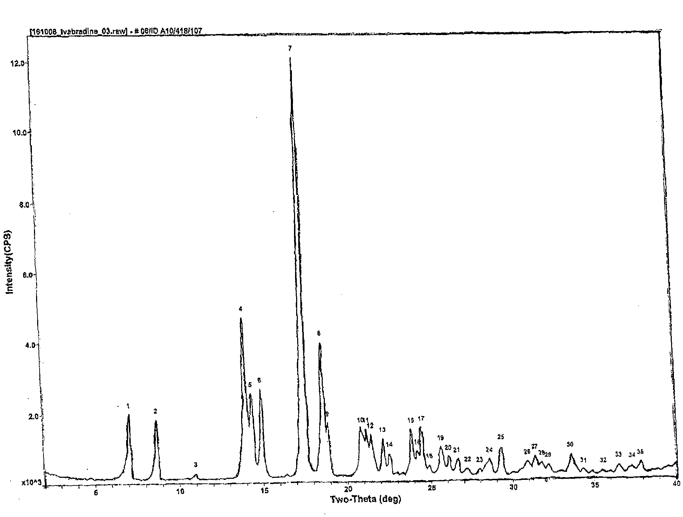

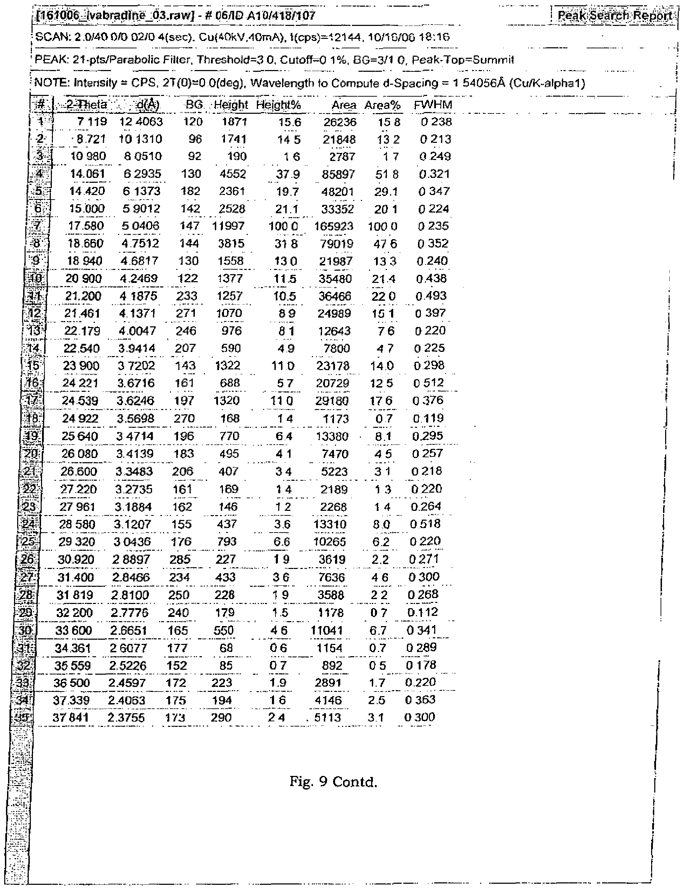

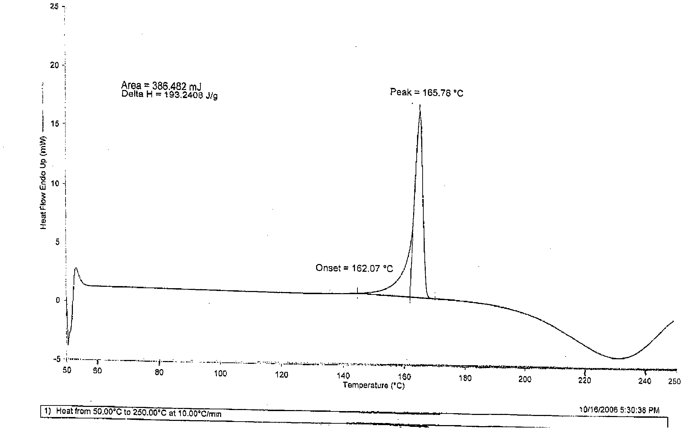

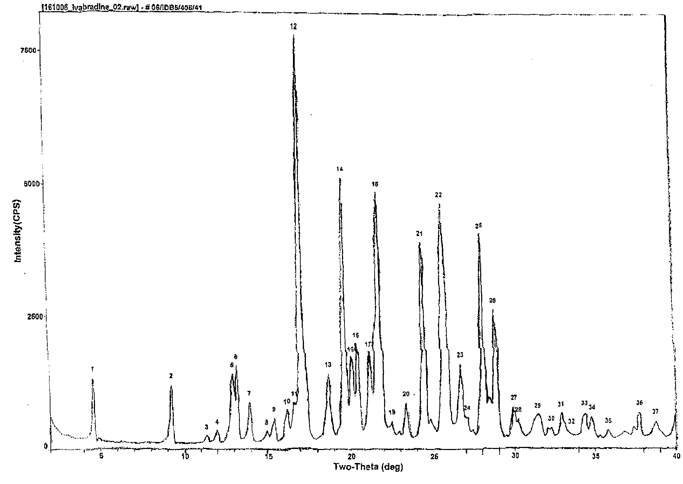

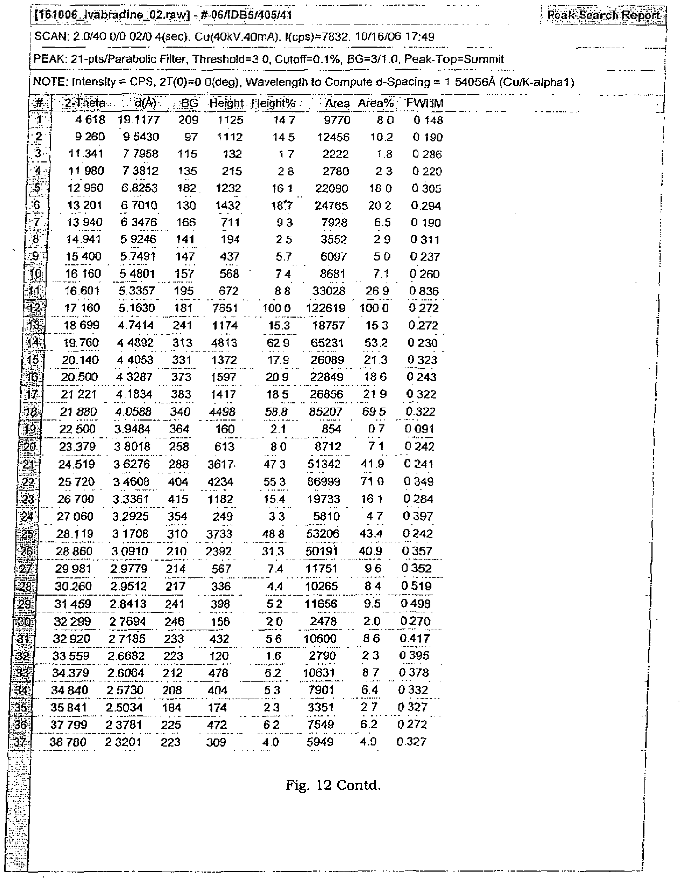

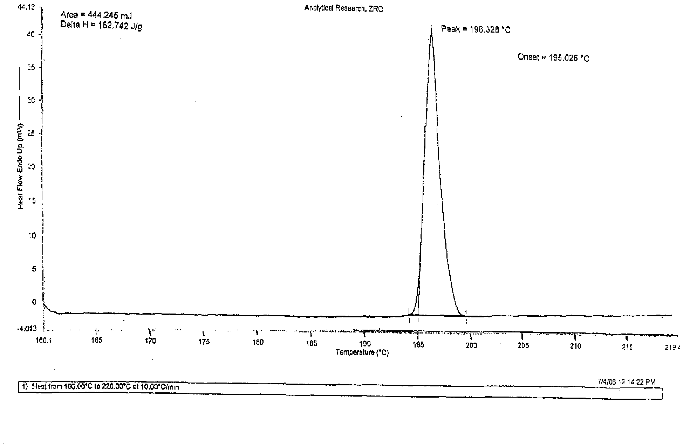

The following constitute particularly preferred numbered embodiments of the present invention:
Numbered Embodiment 1. A process for preparing Ivabradine of formula (I) or its pharmaceutically acceptable salts, solvates, hydrate thereof, 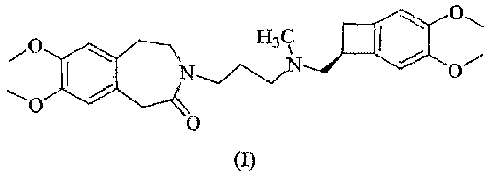
comprising reacting 3-chloro-N-{[(7S)-3,4-dimethoxybicyclo[4.2.0]octa-1,3,5-tnen-7-yl]methyl}-N-methylpropan-1-amine of formula (IV), 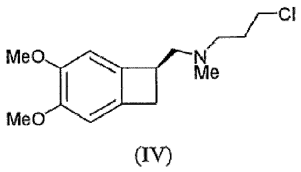
with 7,8-Dimethoxy-1,3,4,5-tetrahydro-benzo[d]azepin-2-one of formula (V) or its alkaline salt 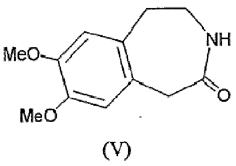 in presence of base in suitable solvent to provide Ivabradine of formula (I), and if desired, converting Ivabridine of formula (I) to its pharmaceutically acceptable salts, solvate or hydrate form.
Numbered Embodiment 2. A process according to Numbered Embodiment 1, wherein said base is selected from sodium hydroxide, potassium hydroxide, sodium carbonate, potassium carbonate, sodium methoxide, sodium ethoxide, potassium tert-butoxide, and sodium hydride.
Numbered Embodiment 3. A process according to Numbered Embodiment 2, wherein the base is potassium carbonate or potassium tert- butoxide.
Numbered Embodiment 4. A process according to Numbered Embodiment 1, wherein suitable solvent is selected from polar protic or aprotic solvents, selected from a lower alcohol (C₁- C₆) such as methanol, ethanol, isopropanol, n-propanol, n-butanol, iso-butanol, tert-butanol; ether such as tetrahydrofuran, diethyl ether, d[upsilon]sopropyl ether, dioxane; sulfoxide such as sulfolane or dimethyl sulfoxide, amides such as dimethyl formamide, dimethyl acetamide, acetonitrile or mixtures thereof.
Numbered Embodiment 5. A process according to Numbered Embodiment 4, wherein said solvent is dimethyl sulfoxide.
Numbered Embodiment 6. A process for preparing Ivabradine of formula (I) or its pharmaceutically acceptable salt, preferably hydrochloride, comprising reacting 3-chloro-N-{[(7S)-3,4-dimethoxybicyclo[4.2.0]octa-1,3,5-trien-7-yl]methyl}-N-methyl propan-1-amine of formula (IV) with 7,8- Dimethoxy-1,3,4,5-tetrahydro-benzo[d] azepin-2-one of formula (V) in presence of base selected from potassium tert-butoxide in dimethylsulfoxide to provide Ivabradine of formula (I).
Numbered Embodiment 7. 3-chloro-N-{[(7S)-3,4-dimethoxybicyclo[4.2.0]octa-1,3, 5-trien-7-yl]methyl}-N- methylpropan-1-amine of formula (IV),. 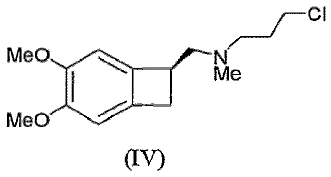
Numbered Embodiment 8. A process for preparing 3-chloro-N-{[(7S)-3,4-dimeth oxybicyclo[4.2.0]octa-1,3,5-trien-7- yl]methyl}-N-methylpropan-1-amine of formula (IV), 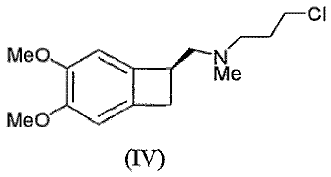 (IV) which comprises reacting 1-[(7S)-3,4-dimethoxybicyclo[4.2.0]octa-1,3,5-trien-7 -yl]-N-methylmethanamine of formula (VI) with 1-Bromo-3-chloropropane of formula (VII) 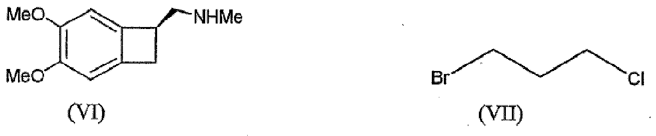 in presence of base to provide 3-chloro-N-{[(7S)-3,4-dimethoxybicyclo[4.2.0]octa-1,3,5- trien-7-yl]methyl}-N-methylpropan-1-amine of formula (IV).
Numbered Embodiment 9. A process according to Numbered Embodiment 8, wherein base is selected from sodium hydroxide, potassium hydroxide, and sodium bicarbonate, preferably potassium bicarbonate.
Numbered Embodiment 10. A process according to Numbered Embodiment 8, wherein said reaction is carried out in solvent selected from lower alcohol (C₁- C₆) such as methanol, ethanol, isopropanol, n-propanol, n-butanol, iso-butanol, tert-butanol; ether such as tetrahydrofuran, diethyl ether, diisopropyl ether, dioxane; sulfoxide such as sulfolane or dimethyl sulfoxide, amides such as dimethyl formamide, dimethyl acetamide.
Numbered Embodiment 11. A process according to Numbered Embodiment 8, wherein said reaction is carried out in neat condition at room temperature.
Numbered Embodiment 12. A crystalline form of (1S)-4,5-dimethoxy-1-(methyl aminomethyl)-benzocyclobutane camphor sulfonate of formula (II), 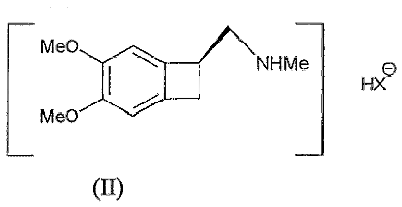 wherein HX = Camphor sulfonic acid
Numbered Embodiment 13. A crystalline salt according to Numbered Embodiment 12, characterized by its X-ray powder diffraction pattern having peaks at 7.1, 8.7, 10.9, 14.0, 14.4, 15.0, 17.5, 18.6, 18.9, 20.9, 21.2, 21.4, 22.1, 22.5, 23.9, 24.2, 24.5, 24.9, 25.6, 26.0, 26.6, 27.2, 27.9, 28.5, 29.3, 30.9, 31.4, 31.8, 32.2, 33.6, 34.3, 35.5, 36.5, 37.3, 37.8 +/- 0.2 two theta values.
Numbered Embodiment 14. A process for preparing novel crystalline form according to Numbered Embodiment 12, which comprises
   (i) reaction of 4,5-dimethoxy-1-(methylaminomethyl)benzocyclobutane with camphor sulfonic acid in suitable organic solvent to form (1S)-4,5-dimethoxy-1-(methylaminomethyl)- benzocyclobutane camphor sulfonate;
   (ii) recrystallizing said salt from solvent selected from methanol, ethanol, n-propanol, isopropanol, n-butanol, isobutanol, tert-butanol, acetone, methyl ethyl ketone, methyl isobutyl ketone or mixtures thereof; and
   (ii) recrystallizing in a suitable organic solvent to form the novel crystalline form.
Numbered Embodiment 15. A novel crystalline form of (1S)-4,5-dimethoxy-1-(methylamine)-benzocyclobutane N-acetyl-L-gluatamic acid of formula (III), 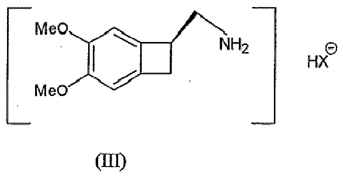 wherein HX = N-acetyl-L-gluatamic acid
Numbered Embodiment 16. A crystalline salt according to Numbered Embodiment 15, characterized by its X-ray powder diffraction pattern having peaks at 8.3, 9.7, 10.7, 12.5, 14.5, 16.0, 16.7, 17.9, 18.7, 19.2, 19.6, 20.4, 21.2, 21.7, 22.7, 23.7, 24.2, 24.6, 25.7, 26.2, 26.7, 28.0, 29.4, 29.8, 30.8, 31.5, 32.4, 33.2, 34.7, 36.0, 36.9, 37.5, 38.3, 39.1 +/- 0.2 two theta values.
Numbered Embodiment 17. A process for preparing novel crystalline form according to Numbered Embodiment 15, which comprises (i) reaction of 1-(3,4-dimethoxybicyclo[4.2.0]octa-1,3,5-trien-7-yl)methanamine with N-acetyl glutamic acid in suitable organic solvent to form 1-(3,4-dimethoxybicyclo [4.2.0]octa-1,3,5-trien-7-yl)methanamine N-acetyl glutamate; and (ii) recrystallizing said salt from solvent selected from methanol, ethanol, n-propanol, isopropanol, n-butanol, isobutanol, tert-butanol, acetone, methyl ethyl ketone, methyl isobutyl ketone or mixtures thereof.
Numbered Embodiment 18. A process according to Numbered Embodiment 8, wherein said 1-[(7S)-3,4-dimethoxybicyclo[4.2.0]octa-1,3,5-trien-7-yl]-N-methyl methanamine (VI) is prepared by converting (1S)-4,5-dimethoxy-1-(methylamine)-benzocyclobutane N-acetyl-L-gluatamic acid of formula (III) to 1-[(7S)-3,4-dimethoxybicyclo[4.2.0]octa-1,3,5-trien-7-yl]methanamine formula (VIII), which upon 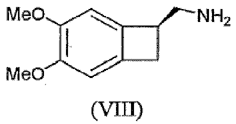 methylation gives 1-[(7S)-3,4-dimethoxybicyclo[4.2.0]octa-1,3,5-trien-7-yl]-N-methylmethanamine (VI) of formula 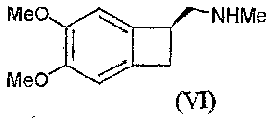
Numbered Embodiment 19. A crystalline form of 7,8-dimethoxy-1,2,3,4-tetra hydro-2H-3-benzapin-2-one of formula (V) 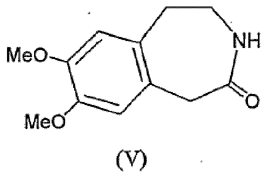 having X-ray powder diffraction pattern with peaks at 4.6, 9.2, 11.3, 11.9, 12.9, 13.2, 13.9, 14.9, 15.4, 16.1, 16.6, 17.1, 18.6, 19.7, 20.1, 20.5, 21.2, 21.8, 22.5, 23.3, 24.5, 25.7, 26.7, 27.0, 28.1, 28.8, 29.9, 30.2, +/- 0.2 two theta values.
Numbered Embodiment 20. A process for preparing novel crystalline form according to Numbered Embodiment 19, which comprises recrystallizing 7,8-dimethoxy-1,2,3,4-tetrahydro-2H-3-benzapin-2-one from ethyl acetate.
Numbered Embodiment 21. Crystalline Ivabradine hydrochloride Form- I.
Numbered Embodiment 22. Crystalline Ivabradine hydrochloride Form- I having X-ray powder diffraction pattern with peaks at 8.6, 9.1, 11.7, 14.6, 17.2, 18.2, 21.5, 23.9, 26.4 and 27.0 +/- 0.2 two theta values.
Numbered Embodiment 23. Crystalline Ivabradine hydrochloride according to
Numbered Embodiment 21, Form- I of Numbered Embodiment 3, further characterized by X-ray powder diffraction peaks at 11.2, 15.3, 19.6, 20.3, 21.0, 22.2, 22.7, 27.7, 29.4 +/- 0.2 two theta values.
Numbered Embodiment 24. Crystalline Ivabradine hydrochloride Form- I according to Numbered Embodiment 21, wherein X-ray powder diffraction pattern is substantially as depicted in Fig. 1.
Numbered Embodiment 25. A process for preparing crystalline form of Ivabradine hydrochloride according to Numbered Embodiment 21, which comprises:
   a) treating Ivabradine with hydrochloric acid in suitable organic solvent;
   b) isolating Ivabradine-, and
   c) recrystallizing Ivabradine hydrochloride from acetonitrile.

## Claims

1. Crystalline Ivabradine Hydrochloride Form-I.

2. Crystalline Ivabradine Hydrochloride Form-I having X-ray powder diffraction pattern with peaks at 8.6, 9.1, 11.7, 14.6, 17.2, 18.2, 21.5, 23.9, 26.4 and 27.0±0.2 two theta values.

3. The crystalline Ivabradine Hydrochloride according to claim 2, wherein Form-I is further **characterized by** X-ray powder diffraction pattern peaks at 11.2, 15.3, 19.6, 20.3, 21.0, 22.2, 22.7, 27.7, 29.4±0.2 two theta values.

4. The crystalline Ivabradine Hydrochloride according to claim 1, wherein Form-I is further **characterized by** X-ray powder diffraction pattern substantially as depicted in Fig. 1.

5. A process for preparing crystalline Ivabradine Hydrochloride Form-I, the process comprises:
a) dissolving Ivabradine in suitable organic solvent;
b) treating with hydrochloric acid; and
c) isolating Ivabradine Hydrochloride.

6. The process according to claim 5, wherein the suitable organic solvent includes lower alcohols C₁-C₄, esters, ketone, ether and the like.

7. The process according to claim 6, wherein the suitable organic solvent includes methanol, ethanol, isopropanol, n-propanol, n-butanol, iso-butanol, tert-butanol, ethyl acetate, isopropyl acetate, butyl acetate, iso-butyl acetate, acetone, methyl ethyl ketone, methyl tert-butyl ketone, tetrahydrofuran, diethyl ether, diisopropyl ether, dioxane and the like.

8. The process according to claim 5, further comprises recrystallizing of Ivabradine Hydrochloride from acetonitrile.
